# EUROPEAN PATENT APPLICATION

(11) **EP 3 111 941 A1**
(43) Date of publication of application: **04.01.2017**
(21) Application number: 15174427.3
(22) Date of filing: 30.06.2015
(51) Int. Cl.: A61K 31/7008, A61K 31/728, A61K 31/737, A61P 19/02

(54) **COMPOSITIONS COMPRISING CHONDROITIN SULFATE FOR PROPHYLAXIS OR TREATMENT OF DAMAGED ARTICULAR CARTILAGE**

(71) Applicant: Patir, Süleyman, Istanbul (TR); Dikmen, Alican, Istanbul (TR); Konukoglu, Osman Burak, Istanbul (TR)
(72) Inventor: Patir, Süleyman, Istanbul (TR)
(74) Representative: Mutlu, Aydin

(57) **Abstract**

The present invention relates to a composition comprising chondroitin sulfate and at least one further active agent selected from hyaluronic acid and N-acetyl glucosamine for treatment of damaged articular cartilage. The composition according to the present invention is characterized by the reduced protein content of the chondroitin sulfate for reducing adverse effects caused by immunologic reactions of the proteins.

## Description

### Technical Field

The present invention relates to the compositions effective for prophylaxis and treatment of joint disorders. More particularly, the present invention pertains to the compositions comprising N-acetyl glucosamine and chondroitin sulfate for prophylaxis and treatment of the diseases associated with damaged articular cartilage.

### Background of the Invention

Glycosaminoglycans are one of the main components of the cartilage matrix and are composed of chondroitin - 4 - and - 6 - sulfate, glucosamine, dermatan sulfate, hyaluronic acid, heparin-heparan sulfate and keratane sulfate polymers *(*Matrix proteoglycans: from molecular design to cellular function, Iozzo R.V., Annu. Rev. Biochem. 1998; 67: 609-52*).* The components of glycosaminoglycans are determined quantitatively in the studies made on healthy knees and knees with osteoarthritis (0-95 age group) of human autopsies by Sven-Olaf Hjertquist and Rudolf Lemperg *(*Identification and concentration of the glycosaminoglycans of human articular cartilage in relation to age and osteoarthritis, Hjertquist S, Lemperg R., Calcified Tissue Research 1972, Volume 10, Issue 1, pp 223-237*).* Chondroitin sulfate in healthy cartilage is found as a mixture of chondroitin - 4 - sulfate and chondroitin - 6 - sulfate in a ratio of 1:1. Chondroitin sulfate is usually found attached to proteins and form chondroitin sulfate proteoglycans which consist of a protein core and a chondroitin sulfate side chain. As getting older, a decrease in the amount of chondroitin sulfate is observed. The reason for this is the insufficient synthesis of proteoglycans by the cartilage chondroitin and the degradation of the former by metalloproteinase enzymes. In the absence of chondroitin sulfate, the nucleus protein synthesis is entirely affected.

Furthermore, as the chondroitin sulfate and glucosamine proteoglycan synthesis are triggered, the cartilage degeneration is prevented *(*In vivo chondroprotection and metabolic synergy of glucosamine and chondroitin sulfate, Lippiello L. et al, Clin. Orthop. Relat. Res. 2000 Dec; (381): 229-40*).* Hyaluronic acid is a polymer composed of N-acetyl glucosamine and glucuronic acid units and the synthesis thereof is dependent on the presence of glucosamine. Hyaluronic acid is present in the synovial liquid in the cartilage and specifies the viscoelasticity of the liquid. In patients with osteoarthritis, there is a decrease in the visco- feature of the synovial liquid and the concentration of hyaluronic acid and a deterioration in the hyaluronic acid molecules.

Hyaluronic acid is one of the widely used preparates in the treatment of knee osteoarthritis. In the academic publications *(*Characterization of the effect of high molecular weight hyaluronan on trans-synovial flow in rabbit knees, Coleman P. J. et al., The Journal of Physiology Volume 514, Issue 1, pages 265-282, January 1999*), (*Efficacy of Intraarticular Hyaluronic Acid Injections in Knee Osteoarthritis, Evanich J. et al., Clinical Orthopaedics & Related Research: September 2001 - Volume 390 - Issue - pp 173-181*), (*Hyaluronic acid in the treatment of osteoarthritis of the knee, Huskisson E.C. et al., Rheumatology (1999) 38 (7): 602-607*)* and the clinical studies *(*Therapeutic effects of hyaluronic acid on osteoarthritis of the knee. A meta-analysis of randomized controlled trials, Wang C.T. et al., J Bone Joint Surg. Am. 2004 Mar;86-A(3):538-45*), (*US-Approved Intra-Articular Hyaluronic Acid Injections are Safe and Effective in Patients with Knee Osteoarthritis: Systematic Review and Meta-Analysis of Randomized, Saline-Controlled Trials, Miller l. E. et al., Clin. Med. Insights Arthritis Musculoskelet. Disord. 2013; 6: 57-63*)* and *(*Intra-Articular Hyaluronic Acid as Treatment in Elderly and Middle-Aged Patients with Knee Osteoarthritis, Uçar D. et al., Open Rheumatol J. 2013; 7: 38-41*)* the protective and therapeutical features thereof are determined in early osteoarthritis and degenerative meniscopathy.

The combination comprising hyaluronic acid and chondroitin sulfate are among the widely used preparates. In the academic publications *(*Effect of chondroitin sulphate in a rabbit model of atherosclerosis aggravated by chronic arthritis, Herrero-beaumont, G. et al., British Journal of Pharmacology Volume 154, Issue 4, pages 843-851, June 2008*)* and *(*Hyaluronic acid in combination with chondroitin sulfate and hyaluronic acid improved the degeneration of synovium and cartilage equally in rabbits with osteoarthritis, Chen, L. et al., Drug Discov. Ther. 2011 Aug;5(4):190-4*),* the protective and therapeutical features thereof in the cartilage destruction, degenerative meniscopathy. In the patent documents US 6,906,044 B2, WO 2013144867 A1 and US 20060135470 A1 the applications used in the treatment of osteoarthritis and cartilage degeneration are mentioned.

In vitro studies showed that the intra-articular use of glucosamine *(Intra-articular delivery of glucosamine for treatment of experimental osteoarthritis created by a medial meniscectomy in a rat model,* Gibson M., et al., J Orthop. Res. 2014 Feb; 32(2): 302-9. Epub 2013 Nov 5*), (*Glucosamine effects in human; a review of effects on glucose metabolism seid effect, safety considerations and efficacy, Anderson J. W. et al., Food and Chemical Toxicology, Volume 43, Issue 2, February 2005, Pages 187-201*)* and N-acetyl glucosamine *(*Chondroprotective activity of N-acetylglucosamine in rabbits with experimental osteoarthritis, Shikhman A.R., et al., Ann. Rheum. Dis. 2005; 64: 89-94*)* are safe and as they trigger the hyaluronic acid synthesis, they prevent also the collagen destruction in chondrocytes.

In patent documents EP 1560588 B1, US 7,485,629 B2, US 7,803,787 B2, US 20130251781 and US 20080003257; intra-articular applications comprising hyaluronic acid, chondroitin sulfate and N-acetyl glucosamine used in the treatment of osteoarthritis and cartilage degeneration. Moreover, in the clinical studies on animals of the intra-articular gel comprising hyaluronic acid, chondroitin sulfate and N-acetyl glucosamine *(*Systemic Effects of Commercial Preparation of chondroitin sulfate, Hyaluronic acid and N-Acetyl-D-glucosamine when Administered Parenteral to Healthy cats, Veir J.K. et al., Inter. J. Appl. Res. Vet. Med. 11(2), 91 -95 (2013*)), (*Evaluation of intra-articular hyaluronan, sodium chondroitin sulfate and N-acetyl-D-glucosamine combination versus saline (0.9% NaCl) for osteoarthritis using an equine model, Frisbie D.D., Vet J. 2013 Sep;197(3):824-9*) and (*Comparison of Synovial Fluid in Middle Carpal Joints Undergoing Needle Aspiration, Infusion with Saline, and Infusion with a Combination of N-Acetyl-d-Glucosamine, Hyaluronic Acid, and Sodium Chondroitin Sulfate, Kawcak, C.E., Journal of Equine Veterinary Science, April 2011 Volume 31, Issue 4, Pages 155-159*),* the therapeutical features thereof are determined.

The chondroitin sulfate used in the market for injection purposes contains high amount of protein as measured based on the protein quantification methods such as Bradford (Bradford MM, (1976) "A rapid and Sensitive Method for the Quantification of Microgram Quantities of Protein Utilizing the Principle of Protein-Dye Binding) or Lowry assay *(*Lowry OH, Rosebrough NJ, Farr AL, Randall RJ (November 1951). "Protein measurement with the Folin phenol reagent". J. Biol. Chem. 193 (1): 265-75*).* The inventors had an attempt to use this product in human with such higher protein content in the form of intra-articular gel, and noted that it causes pain and temporary synovitis by realizing an immunologic reaction in the cartilage on which it is applied. In the course of seeking a solution to these problems, the inventors noted that the reason of adverse events is that protein content, and it is possible to minimize these problems by using chondroitin sulfate with lower protein content. For this reason, an objective of the present invention is to reduce the protein amount of the chondroitin sulfate used in the intra-articular applications.

Another problem encountered in an application comprising chondroitin sulfate and hyaluronic acid and/or N-acetyl glucosamine is the pain that could be caused in the patient after 4 hours of the application. Therefore, minimizing of said pain or completely to eliminate it is a further object of this invention.

### Brief Description of the Invention

The present invention is related to a composition, and preparation thereof, used in the treatment or prophylaxis of damaged articular cartilage such as the case of osteoarthritis and degenerative meniscopathy. Said composition comprises chondroitin sulfate and a further agent selected from hyaluronic acid and N-acetyl glucosamine and. The compositions according the present invention may further comprise at least one anesthetic agent in line with the objectives mentioned above. The composition subject to the current invention, more particularly, is characterized by the reduced protein content of the chondroitin sulfate found in said composition.

The protein amount of chondroitin sulfate found in the composition of the invention is reduced to compatible levels in order to minimize the immunologic reaction that is found to be frequently causing a temporary synovitis. Furthermore, in order to completely eliminate the pain that could be formed within the first 4 hours after the application, a combination with at least one local anesthetic is also realized.

Said anesthetic can be selected from a group comprising tetracaine, amylocaine, bucricaine, bupivacaine, butacaine sulfate, butanilicaine, butoxycaine, carticaine, chloroprocaine, clibucaine, chlormecaine, cyclomethycaine, dimethisoquin hydrochloride, diperodon, diclocaine, ethyl p-piperidine acetylamine benzoate, ethidocaine, hexylcaine, phenacaine, fomocaine, hydroxyprocaine, hydroxytetracaine, ketocaine, oxethazaine, oxybuprocaine, paretoxycaine, piperocaine, piridocaine, pyrrocaine, pramoxine, prilocaine, procaine, propanocaine, propipocaine, propoxycaine, proxymetacaine, ropivacaine, tolycaine, trimecaine, vadocaine agents or any combination thereof.

### Detailed Description of the Invention

The present invention is related to a composition comprising chondroitin sulfate in combination with an agent selected from hyaluronic acid and N-acetyl glucosamine, and optionally at least one local anesthetic. Further aspects of the present invention relate to methods for preparing this composition. Said composition is characterized by the fact that the protein content of chondroitin sulfate in the composition is reduced. The composition prepared according to the invention is preferably an injectable liquid or a gel applicable in animals or human. More preferably, said composition as disclosed herein can be in gel form, and most preferably in the form of a regenerative bio-matrix gel implant.

The prevention of the pain that could be caused through the application of the composition prepared according to the invention in the articulate is provided by the local anesthetic agent found in the composition. Said local anesthetic is selected from a group comprising tetracaine, amylocaine, bucricaine, bupivacaine, butacaine sulfate, butanilicaine, butoxycaine, carticaine, chloroprocaine, clibucaine, chlormecaine, cyclomethycaine, dimethisoquin hydrochloride, diperodon, diclocaine, ethyl p-piperidine acetylamine benzoate, ethidocaine, hexylcaine, phenacaine, fomocaine, hydroxyprocaine, hydroxytetracaine, ketocaine, oxethazaine, oxybuprocaine, paretoxycaine, piperocaine, piridocaine, pyrrocaine, pramoxine, prilocaine, procaine, propanocaine, propipocaine, propoxycaine, proxymetacaine, ropivacaine, tolycaine, trimecaine, vadocaine agents or any combination thereof.

The protein amount of chondroitin sulfate is adjusted such that it is below 0.2 mg, preferably below 0.15 mg, more preferably below 0.1 mg and most preferably below 0.05 mg on 1 g weight basis of chondroitin sulfate, so as not to cause immunologic reactions and temporary synovitis in the joint of the patient. The preferred methods for measuring the aforesaid protein content is either Bradford or Lowry assays whereas Bradford method is particularly preferred. The inventors noted that a protein amount of less than 0.2 mg on 1 g weight basis of chondroitin sulfate substantially eliminates the adverse events mentioned above. According to this, the composition of the invention comprises chondroitin sulfate along with hyaluronic acid and/or N-acetyl glucosamine, and optionally at least one local anesthetic, whereas the composition is characterized by the fact that the protein amount is below 0.2 mg on 1 g weight basis of chondroitin sulfate. Therefore, the inventors have obtained unexpected results in reduction of undesired reactions as mentioned above.

The said protein amount can be reduced by conventional ways as known by those skilled in the art such as chromatography. Therefore, a chromatographic method can be used to reduce the protein amount of chondroitin sulfate in the composition prepared according to the current invention.

The composition of the invention may further comprise at least one pharmaceutically acceptable excipient in addition to active agents. Said excipients can be selected from a group comprising Na₂HPO₄, NaH₂PO₄, NaCl, injectable water and/or combinations thereof.

The molecular weight and the amount of chondroitin sulfate used in the composition of the invention is preferably adjusted for not to cause the immunologic reactions mentioned above, and in order to be able to display therapeutical features. According to this, the molecular weight of the chondroitin sulfate used in the compositions prepared according to the invention is between 10.000 - 30.000 Daltons. In another embodiment, in the compositions of the invention, the amount of the chondroitin sulfate used is between 10 mg and 40 mg.

The molecular weight of hyaluronic acid as one of the active agents used in the composition of the invention ranges from 1.8 to 3 million Daltons. Amount of said active agent preferably ranges in between 12 - 21 mg. The preferred amount of N-acetyl glucosamine in the composition is 10 - 40 mg, while that of local anesthetic is 2 to 6 mg. The local anesthetic used in the composition of the invention is preferably prilocaine, more preferably prilocaine hydrochloride.

In an aspect of the invention, another feature of the composition described above is that the protein content of chondroitin sulfate in said composition is below 0.2 mg, preferably 0.15 mg in 1 g weight of chondroitin sulfate.

In another aspect, the current invention is related to a composition comprising chondroitin sulfate with a protein content of less than 0.2 mg on 1 g weight basis of chondroitin sulfate for use in prophylaxis or treatment of a disease associated with damaged articular cartilage.

In a further embodiment, the present invention is related to a composition comprising hyaluronic acid, N-acetyl glucosamine, chondroitin sulfate with a protein content of less than 0.2 mg on 1 g weight basis of chondroitin sulfate and at least one local anesthetic for use in treatment or prophylaxis of the diseases such as damaged articular cartilage, more particularly of osteoarthritis and degenerative meniscopathy.

In another embodiment, the composition of the present invention is provided in liquid or semi-liquid forms such as injectable liquids or gels. Therefore, in these embodiments where the composition is for use in the prophylaxis or treatment of diseases such as cartilage degeneration in the joints, osteoarthritis, and degenerative meniscopathy, the amount of chondroitin sulfate is 10 mg/ml to 40 mg/ml, the amount of hyaluronic acid is 12 mg/ml to 21 mg/ml, the amount of N-acetyl glucosamine is 10 mg/ml to 40 mg/ml, and if available, the amount of local anesthetic is 2 mg/ml to 6 mg/ml.

In another aspect, the subject-matter of the current invention is related to a method for the preparation of a composition comprising chondroitin sulfate along with an agent selected from hyaluronic acid and N-acetyl glucosamine, and optionally at least one local anesthetic wherein said method is characterized by comprising the step of reducing protein amount in the chondroitin sulfate.

In another embodiment of the invention, the method for the preparation of the aforesaid composition comprises the steps of;
- reducing the protein content of chondroitin sulfate to a level less than 0.2 mg/g, preferably 0.15 mg/g weight of chondroitin sulfate, and
- mixing of chondroitin sulfate with an agent selected from hyaluronic acid and N-acetyl glucosamine.

As noted above, the preferred method for reducing the protein amount is a chromatographic method.

It is possible to develop various types of applications around these main concepts related to the intra-articular compositions of the invention, and the contents of some compositions in intra-articular gel or liquid form that could represent examples for such compositions are given below as exemplary embodiments.

### Examples

### Example 1

| **Compounds** | **Amount (mg/ml)** |
|---|---|
| NaH₂PO₄ | 0.45 |
| Na₂HPO₄ | 2 |
| NaCl | 4.5 |
| Chondroitin sulfate | 30 |
| Hyaluronic acid | 16 |
| N-Acetyl glucosamine | 30 |
| Prilocain | 3 |

### Example 2

| **Compounds** | **Amount (mg/ml)** |
|---|---|
| NaH₂PO₄ | 0.45 |
| Na₂HPO₄ | 2 |
| NaCl | 4.5 |
| Chondroitin sulfate | 30 |
| Hyaluronic acid | 16 |
| Prilocain | 3 |

### Example 3

| **Compounds** | **Amount (mg/ml)** |
|---|---|
| NaH₂PO₄ | 0.45 |
| Na₂HPO₄ | 2 |
| NaCl | 4.5 |
| Chondroitin sulfate | 30 |
| N-Acetyl glucosamine | 30 |
| Prilocain | 3 |

### Example 4

| **Compounds** | **Amount (mg/ml)** |
|---|---|
| NaH₂PO₄ | 0.45 |
| Na₂HPO₄ | 2 |
| NaCl | 4.5 |
| Chondroitin sulfate | 30 |
| Hyaluronic acid | 16 |
| N-Acetyl glucosamine | 30 |

## Claims

1. A composition comprising chondroitin sulfate and at least one additional active agent selected from the group of hyaluronic acid and N-acetyl glucosamine wherein the protein amount of chondroitin sulfate is below 0.2 mg on 1 g weight basis of chondroitin sulfate.

2. The composition according to claim 1 wherein the protein amount of chondroitin sulfate is below 0.15 mg on 1 g weight basis of chondroitin sulfate

3. The composition according to claim 1 wherein said composition comprises chondroitin sulfate, hyaluronic acid and N-acetyl glucosamine.

4. The composition according to any of the claims 1 to 3 wherein the composition further comprises a local anesthetic agent selected from the group consisting of tetracaine, amylocaine, bucricaine, bupivacaine, butacaine sulfate, butanilicaine, butoxycaine, carticaine, chloroprocaine, clibucaine, chlormecaine, cyclomethycaine, dimethisoquin hydrochloride, diperodon, diclocaine, ethyl p-piperidine acetylamine benzoate, ethidocaine, hexylcaine, phenacaine, fomocaine, hydroxyprocaine, hydroxytetracaine, ketocaine, oxethazaine, oxybuprocaine, paretoxycaine, piperocaine, piridocaine, pyrrocaine, pramoxine, prilocaine, procaine, propanocaine, propipocaine, propoxycaine, proxymetacaine, ropivacaine, tolycaine, trimecaine, vadocaine agents or any combination thereof.

5. The composition according to claim 1 wherein the molecular weight of said chondroitin sulfate is between 10.000 - 30.000 Daltons.

6. The composition according to any of the preceding claims wherein said composition is in an injectable liquid or gel form.

7. The composition according to claim 6 wherein the amount of said chondroitin sulfate ranges from 10 mg/ml to 40 mg/ml.

8. The composition according to claim 6 wherein the amount of said hyaluronic acid ranges from 12 mg/ml to 21 mg/ml.

9. The composition according to claim 6 wherein the amount of said N-acetyl glucosamine ranges from 10 mg/ml to 40 mg/ml.

10. The composition according to claim 6 wherein the amount of said local anesthetic ranges from 2 mg/ml to 6 mg/ml.

11. The composition according to claim 4 wherein said local anesthetic is prilocaine.

12. The composition according to claim 1 wherein said composition further comprises an excipient selected from the group consisting of NazHPO₄, NaH₂PO₄, NaCl and injectable water.

13. A composition comprising chondroitin sulfate with a protein content of less than 0.2 mg on 1 g weight basis of chondroitin sulfate, for use in prophylaxis or treatment of a disease associated with damaged articular cartilage.

14. The composition according to claim 13 wherein said disease is selected from osteoarthritis and degenerative meniscopathy.

15. A method for preparing a composition according to claim 1 comprising the steps of:
- reducing the protein content of chondroitin sulfate to a level less than 0.2 mg/g weight of chondroitin sulfate, and
- mixing of chondroitin sulfate with an agent selected from hyaluronic acid and N-acetyl glucosamine.

16. The method according to claim 15 wherein the amount of protein in chondroitin sulfate is reduced by a chromatographic method to a level of less than 0.2 mg/g weight of chondroitin sulfate.

17. The method according to claim 15 wherein the amount of protein in chondroitin sulfate is reduced by a chromatographic method to a level of less than 0.15 mg/g weight of chondroitin sulfate.
